# EUROPEAN PATENT SPECIFICATION

(11) **EP 4 291 140 B1**
(45) Date of publication and mention of the grant of the patent: **16.09.2026**
(21) Application number: 22753084.7
(22) Date of filing: 14.02.2022
(51) Int. Cl.: A61F 2/78

(54) **PROSTHETIC LINERS**
PROTHETISCHE AUSKLEIDUNGEN
REVÊTEMENTS PROTHÉTIQUES

(30) Priority: 15.02.2021 SG 10202101506T
(43) Date of publication of application: 20.12.2023
(73) Proprietor: Tan Tock Seng Hospital Pte Ltd, Singapore 308433 (SG); National Skin Centre (Singapore) Pte Ltd, Singapore 308205 (SG); Singapore University of Technology and Design, Singapore 487372 (SG)
(72) Inventor: BINEDELL, Brian Trevor, Singapore 308433 (SG); TEO, Jia Yee, Singapore 308433 (SG); LOW, Hong Yee, Singapore University of Technology and Design 8 Somapah Road Singapore 487372 (SG); TEY, Hong Liang, Singapore 308205 (SG)
(74) Representative: Barker Brettell LLP
(86) International application number: PCT/SG2022/050070
(87) International publication number: WO 2022/173378

(56) References cited:
- WO-A1-2014/205403
- WO-A2-2009/017762
- US-A1- 2002 040 248
- US-A1- 2013 274 895
- US-A1- 2016 338 858
- US-A1- 2017 367 854
- US-A1- 2018 333 288
- US-B2- 8 808 394
- US-B2- 9 925 072

## Description

### Technical Field

The present disclosure relates generally to prosthetic limbs and prosthetic liners. More particularly, the present disclosure relates to prosthetic limbs and prosthetic liners configured to direct, collect, and/or otherwise control or manage liquid introduced from, by, or as a result of a body part of a user (e.g., sweat) and/or external factors.

### Background

Prosthetic liners are important for wearers of prosthetic limbs to help prevent negative effects from wearing the prosthetics for several hours at a time. Such negative effects may include friction against the hard prosthetic material and moisture buildup from sweat which is not only uncomfortable but may lead to problems such as abrasions, pain, and other skin damage.

Studies have found that more than 53% of prosthetic users feel discomfort due to excessive heat or sweating, and an increment of 1-2°C is sufficient to trigger this discomfort. Conventional prosthetic liners focus on improving fit and comfort by using cushioning materials. However, the human thermoregulatory system may increase the rate of perspiration due to these liners creating a barrier to thermal transfer mechanisms.

US 8 808 394 B2 discloses a prosthetic elastomeric liner, which can be used without lanyards or straps, in which, upon ambulation, perspiration is voided simultaneously with the reestablishment of a vacuum-aided seal without a vacuum pump. The liner's distal tip comprises a buttress anchored sweat port containing a one-way valve continuous with a channel passing through the buttress and liner from its inner surface to its outer surface. The sweat port is connected, optionally integrally, to a prosthetic pin which is inserted into a prosthetic limb.

### Summary

The present disclosure relates generally to prosthetic liners, prosthetics, and/or the like, for addressing conventional problems, including those described above and in the present disclosure, and more specifically, example embodiments relate to improved prosthetic liners, prostheses, and/or the like, including those that improve or reduce discomfort and/or other negative effects normally encountered when using prostheses. For example, embodiments described herein relate to prosthetic liners for use with prostheses.

According to the invention there is provided a prosthetic liner for a prosthetic limb as defined by claim 1. Optional and/or preferable features are defined by the dependent claims. In an exemplary embodiment, a prosthetic limb assembly is described. The prosthetic limb assembly includes a prosthetic limb. The prosthetic limb includes an interior surface forming an interior cavity. The prosthetic limb assembly also includes a prosthetic liner. The prosthetic liner is configured to be housed in the interior cavity of the prosthetic limb. The prosthetic liner includes a liner body. The liner body is formed in a shape resembling a shape of at least a portion of the interior surface of the prosthetic limb. The liner body includes a first end for receiving an insertion of a body part of a user. At least a portion of the first end of the liner body includes a liquid-retentive portion. The liner body also includes a second end opposite to the first end. The liner body also includes an inner surface formed between the first and second ends. The inner surface of the liner body is configured to contact with at least a portion of the body part of the user. The inner surface of the liner body includes a plurality of microchannels. The plurality of microchannels is formed along the inner surface of the liner body. The plurality of microchannels is connected at one end to the liquid-retentive portion of the first end of the liner body. At least one of the plurality of microchannels is configured to receive liquid droplets and direct the received liquid droplets to the liquid-retentive portion of the first end of the liner body. The liner body also includes an outer surface opposite to the inner surface of the liner body. The outer surface of the liner body faces the interior surface of the prosthetic limb and provided in the interior cavity of the prosthetic limb.

In another exemplary embodiment, a prosthetic liner is described. The prosthetic liner is for use with a prosthetic limb. The prosthetic limb includes an interior surface forming an interior cavity. The prosthetic liner includes a liner body. The liner body is formed in a shape resembling a shape of at least a portion of the interior surface of the prosthetic limb. The liner body is configured to be housed in the interior cavity of the prosthetic limb. The liner body includes a first end for receiving an insertion of a body part of a user. At least a portion of the first end of the liner body includes a liquid-retentive portion. The liner body also includes a second end opposite to the first end. The liner body also includes an inner surface formed between the first and second ends. The inner surface of the liner body is configured to contact with at least a portion of the body part of the user. The inner surface of the liner body includes a plurality of microchannels. The plurality of microchannels is formed along the inner surface of the liner body. The plurality of microchannels are connected at one end to the liquid-retentive portion of the first end of the liner body. At least one of the plurality of microchannels is configured to receive liquid droplets and direct the received liquid droplets to the liquid-retentive portion of the first end of the liner body. The liner body also includes an outer surface opposite to the inner surface of the liner body. The outer surface of the liner body faces the interior surface of the prosthetic limb and is provided in the interior cavity of the prosthetic limb.

In another exemplary embodiment, a prosthetic liner is described. The prosthetic liner is for use with a prosthetic limb. The prosthetic limb includes a liner body. The liner body is formed in a shape resembling a shape of at least a portion of the interior surface of the prosthetic limb. The liner body is configured to be housed in the interior cavity of the prosthetic limb. The liner body includes a first end for receiving an insertion of a body part of a user. The liner body also includes a second end opposite to the first end. The liner body also includes an inner surface formed between the first and second ends. The inner surface of the liner body is configured to contact with at least a portion of the body part of the user. The inner surface of the liner body includes a plurality of microchannels. The plurality of microchannels are formed along the inner surface of the liner body. At least one of the plurality of microchannels is configured to receive and collect liquid droplets. The liner body also includes an outer surface opposite to the inner surface of the liner body. The outer surface of the liner body faces the interior surface of the prosthetic limb and is provided in the interior cavity of the prosthetic limb. The outer surface of the liner body includes a plurality of second microchannels. The plurality of second microchannels are formed along the outer surface of the liner body. At least one of the plurality of second microchannels is configured to receive and collect liquid droplets.

### Brief Description of Figures

For a more complete understanding of the present disclosure, example embodiments, and their advantages, reference is now made to the following description taken in conjunction with the accompanying figures, in which like reference numbers indicate like features, and:
**Figure 1** is a top view of an example embodiment of a prosthetic liner for use with a prosthetic limb;
**Figure 2** is a front view of an example embodiment of a prosthetic liner for use with a prosthetic limb; and

Although similar reference numbers may be used to refer to similar elements in the figures for convenience, it can be appreciated that each of the various example embodiments may be considered to be distinct variations.

Example embodiments will now be described with reference to the accompanying figures, which form a part of the present disclosure, and which illustrate example embodiments which may be practiced. As used in the present disclosure and the appended claims, the terms "embodiment," "example embodiment," "exemplary embodiment," and "present embodiment" do not necessarily refer to a single embodiment, although they may, and various example embodiments may be readily combined and/or interchanged without departing from the scope of the invention, which is defined by the appended claims. Examples and embodiments of the description not falling within the scope of the claims do not form part of the invention and are provided for illustrative purposes only. Furthermore, the terminology as used in the present disclosure and the appended claims is for the purpose of describing example embodiments only and is not intended to be limitations. In this respect, as used in the present disclosure and the appended claims, the term "in" may include "in" and "on," and the terms "a," "an," and "the" may include singular and plural references. Furthermore, as used in the present disclosure and the appended claims, the term "by" may also mean "from," depending on the context. Furthermore, as used in the present disclosure and the appended claims, the term "if" may also mean "when" or "upon," depending on the context. Furthermore, as used in the present disclosure and appended claims, the words "and/or" may refer to and encompass any or all possible combinations of one or more of the associated listed items.

### Detailed Description

In the following detailed description, reference is made to the accompanying drawings which form a part hereof. The processes and system described in the detailed description and drawings are for illustrative purposes and are not meant to be limiting. Other embodiments can be utilised and other changes can be made, without departing from the scope of the disclosure presented herein. In the present disclosure, the depiction of a given element or consideration or use of a particular element number in a particular figure or a reference thereto in corresponding descriptive material can encompass the same, an equivalent, or an analogous element or element number identified in another figure or description material associated therewith.

Prosthetic limbs (or prostheses), or the like, play an important part in helping those who have lost a limb to replace at least some functionality and/or appearance of such lost limbs. While prostheses indeed assist in replacing the function and/or appearance of lost limbs, users of prostheses oftentimes encounter varying degrees of discomfort and/or other negative effects resulting from the use of prostheses. For example, studies have found that more than 53% of prosthetic users encounter discomfort and/or other negative effects due as a result of heat or sweating, and an increment of merely 1-2°C is sufficient to trigger this discomfort.

Present example embodiments relate generally to and/or include prosthetic liners, prostheses, and/or the like, for addressing conventional problems, including those described above and in the present disclosure, and more specifically, example embodiments relate to improved prosthetic liners, prostheses, and/or the like, including those that improve or reduce discomfort and/or other negative effects normally encountered when using prostheses. For example, embodiments described herein relate to prosthetic liners for use with prostheses.

It is to be understood that, while example embodiments are mostly described in the present disclosure as pertaining to prosthetic liners, the principles described in the present disclosure may also be applied beyond the context of prosthetic liners for use with prosthetics, such as liners for use with other wearable objects, gear, devices, or the like, and prostheses having liners formed as a unitary article, without departing from the teachings of the present disclosure.

Example embodiments will now be described below with reference to the accompanying figures, which form a part of the present disclosure.

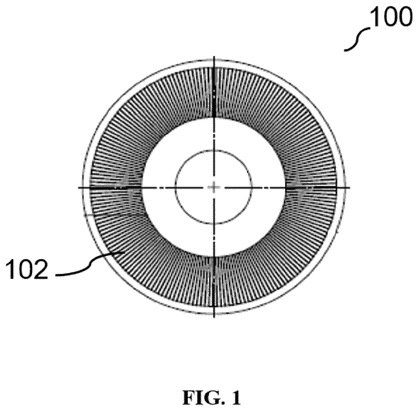

**FIGURE 1** illustrates an example embodiment of a prosthetic liner (e.g., prosthetic liner 100). The prosthetic liner 100 may be configurable or configured for use with a prosthetic limb (or prostheses, not shown) to address one or more conventional problems described above and in the present disclosure. For example, the prosthetic liner 100 may be provided in, provided on, and/or attached or secured to at least a part of a prosthetic in such a way that, when in use, the prosthetic liner 100 is adjacent to and/or contacts with a body part of a user. As a more specific but non-limiting example, the prosthetic liner 100 may be provided in, provided on, and/or attached or secured to an inner surface of a prosthetic arm, prosthetic leg, or the like.

An example embodiment of the prosthetic liner 100 may be formed in any one or more shapes and using any one or more types of materials that provide for an improvement to the fit and comfort of wearing the prosthetics. It is recognized in the present disclosure, however, that the human thermoregulatory system may increase the rate of perspiration when using prosthetics and/or prosthetic liners, which may result in creating a barrier, layer, or the like, to thermal transfer mechanisms and causing discomfort and/or other negative effects. Furthermore, friction against the prosthetic material, constant contact against one or more surfaces of the prosthetics, and/or continued sweat and/or moisture buildup may cause further discomfort and/or other negative effects, including abrasions, pain, skin rashes, and/or other skin damage. While various solutions and approaches may be used to address such discomfort and/or other negative effects users face when using prosthetics, such as the use of powders, creams, or the like, to protect the skin of prosthetic users, it is recognized in the present disclosure that such approaches remain insufficient, especially when addressing the problem of sweat and/or moisture buildup resulting from extended use of prosthetics (e.g., wearing of prosthetics for several hours at a time).

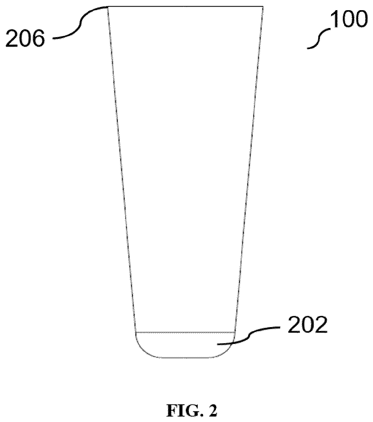

In an example embodiment, the prosthetic liner 100 can be formed in the shape resembling an interior of the prosthetic and a size suitable for the prosthetic liner 100 to be provided in, provided on, and/or attached or secured to the prosthetic and for the prosthetic liner 100 to be adjacent to and/or in contact with a body part of the user. As illustrated in **FIGURE 2****,** the prosthetic liner 100 includes a top section (e.g., top section 206), which may be formed as or having an open end (for insertion of a body part of the user). The prosthetic liner 100 may also include a bottom section (e.g., bottom section 202), which may be formed as a closed end. In some embodiments, the bottom section 202 may also be formed as an open or partially open end.

The prosthetic liner 100 also includes one or more side walls, or the like, formed between and secured to the top section 206 and bottom section 202 of the prosthetic liner 100. For example, a single continuous side wall may be formed between the top section 206 and bottom section 202, such as in example embodiments in which the prosthetic liner is formed in the shape of a cylinder, or the like. Each of the one or more side walls of the prosthetic liner 100 includes an inner surface directly facing, adjacent to, and/or in contact with a body part of the user. Furthermore, each inner surface of the prosthetic liner 100 includes at least a portion having a plurality of microchannels (e.g., microchannels 102), or the like. Furthermore, an inner surface of the bottom section 202 (e.g., when the bottom section 202 is formed as a closed end) may also include at least a portion having a plurality of the microchannels 102. As will be further described in the present disclosure, example embodiments of the microchannels 102 are configured to collect, direct, and/or otherwise control or manage liquid (e.g., sweat, moisture, water, etc.) introduced from, by, or as a result of the body part of the user and/or external factors (e.g., environment, humidity, etc.). Figure 1 illustrates an example embodiment in which the entire inner surface of the side walls of the prosthetic liner 100 includes microchannels 102. In such a case, the prosthetic liner 100 may (or may not) be formed as a unitary article (e.g., a unitary cylindrical or other shaped liner having microchannels 102 formed throughout the entire inner surface, etc.). It is to be understood in the present disclosure, however, that in some embodiments, the prosthetic liner 100 may be formed in such a way that one or more portions of the inner surface of the prosthetic liner 100 may not include microchannels 102. For example, the prosthetic liner 100 may be formed via a plurality of quadrilateral shaped pieces (and/or other geometric shaped pieces), with at least one piece having microchannels 102 (e.g., for parts of the body part of the user that contacts with or is expected to contact with the prosthetic liner 100, etc.) and at least one piece not having microchannels 102 (e.g., for parts of the body part of the user that do not contact with or is not expected to contact with the prosthetic liner 100, etc.).

Each of the one or more side walls of the prosthetic liner 100 also includes an outer surface facing the opposite direction as the inner surface of the prosthetic liner 100. In some embodiments, the inner surface and outer surface are formed as separate walls, or the like. Each outer surface (or outer wall) of the prosthetic liner 100 may include at least a portion having a plurality of microchannels (e.g., microchannels 102), or the like. Furthermore, an outer surface of the bottom section 202 (e.g., when the bottom section 202 is formed as a closed end) may also include a portion having a plurality of the microchannels 102. As will be further described in the present disclosure, example embodiments of the microchannels 102 are configured to collect, direct, and/or otherwise control liquid (e.g., sweat, moisture, water, etc.) between the prosthetic liner 100 and the prosthetic limb coming from outside of the prosthetic limb. In example embodiments, the entire outer surface of the side walls of the prosthetic liner 100 may include microchannels 102. In such a case, the prosthetic liner 100 may (or may not) be formed as a unitary article (e.g., a unitary cylindrical or other shaped liner having microchannels 102 formed throughout the entire outer surface, etc.). It is to be understood in the present disclosure, however, that in some embodiments, the prosthetic liner 100 may be formed in such a way that one or more portions of the outer surface of the prosthetic liner 100 may not include microchannels 102. For example, the prosthetic liner 100 may be formed via a plurality of quadrilateral shaped pieces (and/or other geometric shaped pieces), with at least one piece having microchannels 102 formed on the outer surface and at least one piece not having microchannels 102 formed on the outer surface.

In an example embodiment, the microchannels 102 of the inner surface and/or outer surface of the prosthetic liner 100 may be formed using any one or more types/forms/compositions of soft hydrophilic material, including those having polyurethane-like properties. In an example embodiment, some, or all of the microchannels 102 of the inner surface and/or outer surface of the prosthetic liner 100 may be formed from materials having a shore hardness of about 40A. In an example embodiment, a dimension of each microchannel 102 may be not more than about 0.2 mm (e.g., a diameter or width of not more than about 0.2 mm).

In use, the prosthetic liner 100 may be rolled on from the bottom section 202 to the top section 206. The inner surface of the prosthetic liner 100 having microchannels 102 formed on at least a portion of the inner surface will be in contact with the skin of the user. The prosthetic liner 100 may be removed by unrolling the prosthetic liner 100 from the top section 206 to the bottom section 202 and can be repeatedly washed like regular laundry.

During use, a wearer of the prosthetic liner 100 will secrete sweat throughout the day. The microchannels 102 of the inner surface (and in some embodiments, the outer surface as well) of the prosthetic liner 100 receive and collect liquid droplets (e.g., sweat droplets) secreted by the user. Due to the low surface tension of the material of microchannels 102, forces of adhesion introduced by the microchannels 102 are greater than other forces, including those pertaining to cohesion and gravity. In this regard, the liquid droplets will effectively cling to the interior walls of the microchannels 102. Furthermore, the upper surface of each liquid droplet (i.e., liquid-air interface) spreads out in a concave-like shape in which the height of the liquid droplet at the point of contact with the interior walls of the microchannels 102 is higher than liquid fluids at the middle of the microchannels 102. While the cohesion force between liquid molecules within a liquid droplet will tend to attract to each other, it is recognized in the present disclosure that the liquid molecules will be directed along, drawn, and/or "climb" (e.g., upwards or against gravity) the microchannels 102 at a steady rate (e.g., where both adhesion and cohesion forces are equal). In preferred embodiments, the microchannels 102 will be formed in such a way that the liquid droplets will be directed upwards (e.g., in a direction from the bottom section 202 to the top section 206 of the prosthetic liner 100). It is to be understood, however, that the microchannels 102 may be formed in other ways and/or orientations so as to direct liquid droplets in other directions without departing from the teachings of the present disclosure.

In example embodiments in which the microchannels 102 direct liquid upwards, at least a portion of the top section 206 of the prosthetic liner 100 may include a liquid-retentive portion. The liquid-retentive portion may be formed using and/or having liquid absorbent and/or liquid retentive material, or the like, which may function to collect liquid (e.g., sweat, moisture, etc.) which has been collected by and travelled along the microchannels 102 throughout the day. For example, some, most, or all of the top section 206 of the prosthetic liner 100 may include liquid absorbent or retentive fabric, polymers, or the like (e.g., super absorbent polymers, or SAPs). In other embodiments, the liquid-retentive portion may be provided in other parts of the prosthetic liner 100, such as near the bottom section 202 of the prosthetic liner 100 and/or at one or more locations between the top section 206 and the bottom section 202 of the prosthetic liner 100.

In some embodiments, other suitable methods may be used to direct liquid towards the liquid-retentive portion (which may be provided in the top section 206, bottom section 202, and/or other parts of the prosthetic liner 100). Such suitable methods may include, but are not limited to, the use of pumps, negative pressure (e.g., vacuum, valves), other hydrophilic materials, formations, and/or assemblies, or the like.

As another example, for prosthetic limbs that regularly come into contact with other body parts (e.g., prosthetic limbs for arms or legs that come into contact with one or more other body parts when the user bends or moves a joint (e.g., an elbow, shoulder, or knee (each as applicable))), the portion(s) of the top section 206 of the prosthetic liner 100 that regularly come into contact with other body parts may not include liquid absorbing or retentive material since such contact with other body parts may cause collected liquids to be released. It is recognized in the present disclosure that such an embodiment prevents moisture buildup within and/or at the bottom section 202 of the prosthetic liner, which would otherwise cause discomfort and other negative effects to the user.

While various aspects and embodiments have been disclosed herein, it will be appreciated by a person skilled in the art that several of the above-disclosed structures, parameters, or processes thereof, can be desirably modified, adapted, and combined into alternative structures, processes and/or applications. The various aspects and embodiments disclosed herein are for purposes of illustration and are not intended to be limiting, with the true scope of the disclosure being indicated by the claims.

Various terms used herein have special meanings within the present technical field. Whether a particular term should be construed as such a "term of art" depends on the context in which that term is used. Terms are to be construed in light of the context in which they are used in the present disclosure and as one of ordinary skill in the art would understand those terms in the disclosed context. Definitions provided herein are not exclusive of other meanings that might be imparted to those terms based on the disclosed context.

Words of comparison, measurement, and timing such as "at the time", "equivalent", "during", "complete", and the like should be understood to mean "substantially at the time", "substantially equivalent", "substantially during", "substantially complete", etc., where "substantially" means that such comparisons, measurements, and timings are practicable to accomplish the implicitly or expressly stated desired result.

Additionally, the section headings and topic headings herein are provided for consistency with the suggestions under various patent regulations and practice, or otherwise to provide organizational cues. These headings shall not limit or characterize the embodiments set out in any claims that may issue from this disclosure. Specifically, a description of a technology in the "Background" is not to be construed as an admission that technology is prior art to any embodiments in this disclosure. Furthermore, any reference in this disclosure to "invention" in the singular should not be used to argue that there is only a single point of novelty in this disclosure. Multiple inventions may be set forth according to the limitations of the claims issuing from this disclosure, and such claims accordingly define the invention(s), and their equivalents, that are protected thereby. **In** all instances, the scope of such claims shall be considered on their own merits in light of this disclosure but should not be constrained by the headings herein.

## Claims

1. A prosthetic liner (100) for a prosthetic limb having an interior surface forming an interior cavity, the prosthetic liner (100) comprising:
a liner body, the liner body formed in a shape resembling a shape of at least a portion of the interior surface of the prosthetic limb, the liner body having a top section (206) for receiving an insertion of a body part of a user and a bottom section (202) opposite to the top section (206), **characterized in that** the liner body includes:
a liquid-retentive portion formed in the top section (206) of the liner body, the liquid-retentive portion configured to collect liquid, the liquid-retentive portion formed using liquid retentive materials; and
a plurality of microchannels (102) formed along an inner surface of the liner body, the plurality of microchannels (102) connected at one end to the liquid-retentive portion, wherein each microchannel (102) is formed with soft hydrophilic material in such a way that, when the microchannel (102) receives a liquid droplet, the microchannel (102) is configured to exert a force on the liquid droplet so as to direct the liquid droplet against gravity to the liquid-retentive portion.

2. The prosthetic liner (100) according to claim 1, wherein the liquid-retentive portion includes super absorbent polymers.

3. The prosthetic liner (100) according to claim 1, wherein the liner body is configured to be housed in the interior cavity of the prosthetic limb.

4. The prosthetic liner (100) according to claim 1, wherein the liner body further includes:
an inner surface formed between the top section (206) and bottom section (202), the inner surface of the liner body for contacting with at least a portion of the body part of the user.

5. The prosthetic liner (100) according to claim 1, wherein the liner body further includes an outer surface opposite to the inner surface of the liner body, the outer surface of the liner body being configured for facing the interior surface of the prosthetic limb and be provided in use in the interior cavity of the prosthetic limb.

6. The prosthetic liner (100) according to claim 1, wherein at least one of the following apply:
each of the plurality of microchannels (102) has a width of not more than 0.2 mm; and
each of the plurality of microchannels (102) is formed from materials having a shore hardness of at least 40A.

7. The prosthetic liner (100) according to claim 1, wherein at least a portion of the bottom section (202) of the liner body includes a second liquid-retentive portion.

8. The prosthetic liner (100) according to claim 7, wherein the second liquid-retentive portion is formed using liquid absorbent or retentive materials.

9. The prosthetic liner (100) according to claim 8, wherein the second liquid-retentive portion includes super absorbent polymers.

10. The prosthetic liner (100) according to claim 7, wherein:
at least one of the plurality of microchannels (102) is connected at another end to the second liquid-retentive portion; and
the at least one of the plurality of microchannels (102) that is connected at the another end to the second liquid-retentive portion is configured to receive liquid droplets and direct the received liquid droplets to the second liquid-retentive portion.

11. The prosthetic liner according to claim 5, wherein the outer surface of the liner body includes:
a plurality of second microchannels (102) formed along the outer surface of the liner body, the plurality of second microchannels (102) connected at one end to the liquid-retentive portion, wherein each second microchannel (102) is formed with soft hydrophilic material in such a way that, when the second microchannel (102) receives a second liquid droplet, the second microchannel (102) is configured to exert a force on the second liquid droplet so as to direct the second liquid droplet against gravity to the liquid-retentive portion.

## Patentansprüche

1. Prothetische Auskleidung (100) für eine prothetische Gliedmaße, die eine Innenfläche aufweist, die einen Innenhohlraum ausbildet, wobei die prothetische Auskleidung (100) Folgendes umfasst:
einen Auskleidungskörper, wobei der Auskleidungskörper in einer Form ausgebildet ist, die einer Form mindestens eines Bereichs der Innenfläche der prothetischen Gliedmaße ähnelt, wobei der Auskleidungskörper einen oberen Abschnitt (206) zum Aufnehmen einer Einführung eines Körperteils eines Benutzers und einen unteren Abschnitt (202) gegenüber dem oberen Abschnitt (206) aufweist, **dadurch gekennzeichnet, dass** der Auskleidungskörper Folgendes beinhaltet:
einen flüssigkeitszurückhaltenden Bereich, der in dem oberen Abschnitt (206) des Auskleidungskörpers ausgebildet ist, wobei der flüssigkeitszurückhaltende Bereich dazu konfiguriert ist, Flüssigkeit zu sammeln, wobei der flüssigkeitszurückhaltende Bereich unter Verwendung von flüssigkeitszurückhaltenden Materialien ausgebildet ist; und
eine Vielzahl von Mikrokanälen (102), die entlang einer Innenfläche des Auskleidungskörpers ausgebildet ist, wobei die Vielzahl von Mikrokanälen (102) an einem Ende mit dem flüssigkeitszurückhaltenden Bereich verbunden ist, wobei jeder Mikrokanal (102) derart mit weichem hydrophilen Material ausgebildet ist, dass, wenn der Mikrokanal (102) ein Flüssigkeitströpfchen aufnimmt, der Mikrokanal (102) dazu konfiguriert ist, eine Kraft auf das Flüssigkeitströpfchen auszuüben, um das Flüssigkeitströpfchen gegen die Schwerkraft zu dem flüssigkeitszurückhaltenden Bereich zu leiten.

2. Prothetische Auskleidung (100) nach Anspruch 1, wobei der flüssigkeitszurückhaltende Bereich superabsorbierende Polymere beinhaltet.

3. Prothetische Auskleidung (100) nach Anspruch 1, wobei der Auskleidungskörper dazu konfiguriert ist, in dem Innenhohlraum der prothetischen Gliedmaße untergebracht zu werden.

4. Prothetische Auskleidung (100) nach Anspruch 1, wobei der Auskleidungskörper ferner Folgendes beinhaltet:
eine Innenfläche, die zwischen dem oberen Abschnitt (206) und dem unteren Abschnitt (202) ausgebildet ist, wobei die Innenfläche des Auskleidungskörpers zum Kontaktieren mit mindestens einem Bereich des Körperteils des Benutzers dient.

5. Prothetische Auskleidung (100) nach Anspruch 1, wobei der Auskleidungskörper ferner eine Außenfläche beinhaltet, die der Innenfläche des Auskleidungskörpers gegenüberliegt, wobei die Außenfläche des Auskleidungskörpers dazu konfiguriert ist, der Innenfläche der prothetischen Gliedmaße zugewandt zu sein und bei Verwendung in dem Innenhohlraum der prothetischen Gliedmaße bereitgestellt zu sein.

6. Prothetische Auskleidung (100) nach Anspruch 1, wobei mindestens eines von Folgendem gilt:
jeder der Vielzahl von Mikrokanälen (102) weist eine Breite von nicht mehr als 0,2 mm auf; und
jeder der Vielzahl von Mikrokanälen (102) wird aus Materialien ausgebildet, die eine Shore-Härte von mindestens 40A aufweisen.

7. Prothetische Auskleidung (100) nach Anspruch 1, wobei mindestens ein Bereich des unteren Abschnitts (202) des Auskleidungskörpers einen zweiten flüssigkeitszurückhaltenden Bereich beinhaltet.

8. Prothetische Auskleidung (100) nach Anspruch 7, wobei der zweite flüssigkeitszurückhaltende Bereich unter Verwendung von flüssigkeitsabsorbierenden oder -zurückhaltenden Materialien ausgebildet ist.

9. Prothetische Auskleidung (100) nach Anspruch 8, wobei der zweite flüssigkeitszurückhaltende Bereich superabsorbierende Polymere beinhaltet.

10. Prothetische Auskleidung (100) nach Anspruch 7, wobei:
mindestens einer der Vielzahl von Mikrokanälen (102) an einem anderen Ende mit dem zweiten flüssigkeitszurückhaltenden Bereich verbunden ist; und
der mindestens eine der Vielzahl von Mikrokanälen (102), der an dem anderen Ende mit dem zweiten flüssigkeitszurückhaltenden Bereich verbunden ist, dazu konfiguriert ist, Flüssigkeitströpfchen aufzunehmen und die aufgenommenen Flüssigkeitströpfchen zu dem zweiten flüssigkeitszurückhaltenden Bereich zu leiten.

11. Prothetische Auskleidung nach Anspruch 5, wobei die Außenfläche des Auskleidungskörpers Folgendes beinhaltet:
eine Vielzahl von zweiten Mikrokanälen (102), die entlang der Außenfläche des Auskleidungskörpers ausgebildet ist, wobei die Vielzahl von zweiten Mikrokanälen (102) an einem Ende mit dem flüssigkeitszurückhaltenden Bereich verbunden ist, wobei jeder zweite Mikrokanal (102) derart mit weichem hydrophilen Material ausgebildet ist, dass, wenn der zweite Mikrokanal (102) ein zweites Flüssigkeitströpfchen aufnimmt, der zweite Mikrokanal (102) dazu konfiguriert ist, eine Kraft auf das zweite Flüssigkeitströpfchen auszuüben, um das zweite Flüssigkeitströpfchen gegen die Schwerkraft zu dem flüssigkeitszurückhaltenden Bereich zu leiten.

## Revendications

1. Manchon prothétique (100) pour un membre prothétique présentant une surface intérieure formant une cavité intérieure, le manchon prothétique (100) comprenant :
un corps de manchon, le corps de manchon étant façonné selon une géométrie s'apparentant à la forme d'au moins une portion de la surface intérieure du membre prothétique, le corps de manchon présentant une section supérieure (206) pour recevoir l'insertion d'une partie du corps d'un utilisateur et une section inférieure (202) opposée à la section supérieure (206), **caractérisé en ce que** le corps de manchon comprenne :
une portion de rétention de liquide formée dans la section supérieure (206) du corps de manchon, la portion de rétention de liquide étant configurée pour collecter un liquide, la portion de rétention de liquide étant formée en utilisant des matériaux de rétention de liquide ; et
une pluralité de microcanaux (102) formés le long d'une surface interne du corps de manchon, la pluralité de microcanaux (102) étant reliés par une première extrémité à la portion de rétention de liquide, dans lequel chaque microcanal (102) est formé avec un matériau hydrophile souple de sorte que, lorsque le microcanal (102) reçoit une gouttelette de liquide, le microcanal (102) soit configuré pour exercer une force sur la gouttelette de liquide afin de diriger la gouttelette de liquide à l'encontre de la gravité vers la portion de rétention de liquide.

2. Manchon prothétique (100) selon la revendication 1, dans lequel la portion de rétention de liquide comprend des polymères superabsorbants.

3. Manchon prothétique (100) selon la revendication 1, dans lequel le corps de manchon est configuré pour être logé dans la cavité intérieure du membre prothétique.

4. Manchon prothétique (100) selon la revendication 1, dans lequel le corps de manchon comprend en outre :
une surface interne formée entre la section supérieure (206) et la section inférieure (202), la surface interne du corps de manchon étant destinée à se mettre en contact avec au moins une portion de la partie du corps de l'utilisateur.

5. Manchon prothétique (100) selon la revendication 1, dans lequel le corps de manchon comprend en outre une surface externe opposée à la surface interne du corps de manchon, la surface externe du corps de manchon étant configurée pour être tournée vers la surface intérieure du membre prothétique et être disposée, lors de l'utilisation, dans la cavité intérieure du membre prothétique.

6. Manchon prothétique (100) selon la revendication 1, dans lequel au moins l'une des conditions suivantes s'applique :
chacun de la pluralité de microcanaux (102) présente une largeur n'excédant pas 0,2 mm ; et
chacun de la pluralité de microcanaux (102) est formé à partir de matériaux présentant une dureté shore d'au moins 40A.

7. Manchon prothétique (100) selon la revendication 1, dans lequel au moins une portion de la section inférieure (202) du corps de manchon comprend une seconde portion de rétention de liquide.

8. Manchon prothétique (100) selon la revendication 7, dans lequel la seconde portion de rétention de liquide est formée en utilisant des matériaux d'absorption ou de rétention de liquide.

9. Manchon prothétique (100) selon la revendication 8, dans lequel la seconde portion de rétention de liquide comprend des polymères superabsorbants.

10. Manchon prothétique (100) selon la revendication 7, dans lequel :
au moins un de la pluralité de microcanaux (102) est relié par une autre extrémité à la seconde portion de rétention de liquide ; et
l'au moins un de la pluralité de microcanaux (102) qui est relié par l'autre extrémité à la seconde portion de rétention de liquide est configuré pour recevoir des gouttelettes de liquide et diriger les gouttelettes de liquide reçues vers la seconde portion de rétention de liquide.

11. Manchon prothétique selon la revendication 5, dans lequel la surface externe du corps de manchon comprend :
une pluralité de seconds microcanaux (102) formés le long de la surface externe du corps de manchon, la pluralité de seconds microcanaux (102) étant reliés par une première extrémité à la portion de rétention de liquide, dans lequel chaque second microcanal (102) est formé avec un matériau hydrophile souple de sorte que, lorsque le second microcanal (102) reçoit une seconde gouttelette de liquide, le second microcanal (102) soit configuré pour exercer une force sur la seconde gouttelette de liquide afin de diriger la seconde gouttelette de liquide à l'encontre de la gravité vers la portion de rétention de liquide.
